# EUROPÄISCHE PATENTSCHRIFT

(11) **EP 0 898 979 B1**
(45) Veröffentlichungstag und Bekanntmachung des Hinweises auf die Patenterteilung: **31.03.2004**
(21) Anmeldenummer: 98111554.6
(22) Anmeldetag: 23.06.1998
(51) Int. Cl.: A61M 16/18

(54) **Verriegelungsanordnung für Verdampfer bei einem Narkosegerät**
Pre-regulating device for an anaesthetic vaporiser
Dispositif de préréglage d'un vaporisateur

(30) Priorität: 21.08.1997 SE 9703015
(43) Veröffentlichungstag der Anmeldung: 03.03.1999
(73) Patentinhaber: Maquet Critical Care AB, 171 95 Solna (SE)
(72) Erfinder: Kronekvist, Hans, 115 37 Stockholm (SE)
(74) Vertreter: Samzelius, Roger Mikael

(56) Entgegenhaltungen:
- EP-A- 0 860 176
- EP-A2- 0 376 649
- US-A- 4 058 120
- US-A- 4 307 718
- US-A- 4 434 790

## Beschreibung

Die Erfindung betrifft eine Verriegelungsanordnung für Verdampfer bei einem Narkosegerät, bei dem das Gerät mindestens zwei Verdampfer zum Einstellen einer gewünschten Gaskonzentration umfaßt, wobei die Verriegelungsanordnung Mittel aufweist, die dazu vorgesehen sind zu verhindern, daß mehr als ein Verdampfer gleichzeitig aktiviert wird, wobei die Verdampfer mit einem Gasverteiler verbindbar sind.

Die Erfindung bezieht sich insbesondere auf eine Verriegelungsanordnung für Verdampfer bei einem Narkosegerät, bei dem das Gerät mindestens zwei Verdampfer zum Einstellen einer gewünschten Gaskonzentration und ein zu jedem Verdampfer dazugehörendes Ventil umfaßt, wobei das Ventil den Gasfluß durch den jeweiligen Verdampfer steuert, sowie Mittel, die dazu vorgesehen sind, ein Öffnen und ein Schließen der Ventile zu ermöglichen und die gleichzeitig dazu vorgesehen sind zu verhindern, daß mehr als ein Verdampfer aktiviert wird, wobei die Verdampfer und die Ventile mit einem Gasverteiler verbindbar sind.

Ein Narkosegerät der zuletzt genannten Art, das mit einem Verdampferkarussell versehen ist, ist im Dokument EP-A-0 860 176, Stand der Technik Laut Art. 54(3) EPÜ, näher beschrieben. Das Verdampferkarussell umfaßt hier den Gasverteiler bzw. die Halterung für die genannten Verdampfer und Ventile und eine Gasversorgungseinheit, wobei der Gasverteiler im Verhältnis zur Gasversorgungseinheit rotierbar ist. Es ist hierdurch für einen Arzt einfach, die Position gegenüber dem Patienten zu wechseln und trotzdem eine volle Kontrolle darüber zu haben, welcher Verdampfer aktiviert ist und welche Narkosemittelkonzentration an dem aktiven Verdampfer eingestellt worden ist. Bei Bedarf kann der Narkosearzt, trotz Wechseln der Arbeitsposition, die Konzentration des Narkosemittels ändern, indem er den Gasverteiler bzw. die Halterung dreht, so daß der aktivierte Verdampfer eine gute Position für eine solche Änderung der eingestellten Konzentration erhält. Um zu vermeiden, daß mehr als ein Verdampfer gleichzeitig aktiviert wird, ist in der Anmeldung erwähnt worden, daß eine Verriegelungsanordnung vorhanden ist. Es ist in der Anmeldung nicht näher beschrieben, wie eine solche Verriegelungsanordnung ausgebildet ist.

In der US-PS 4 307 718 ist eine Verriegelungsanordnung für zwei nebeneinander angeordnete Verdampfer für ein Narkosegerät beschrieben. Jeder Verdampfer umfaßt einen zylindrischen Behälter mit einem als Deckel daraufliegenden Einstellrad zum Einstellen der Dampfkonzentration. Jedes Einstellrad weist an dessen zylindrischer Peripherieoberfläche eine Ausnehmung auf. Die Verriegelungsanordnung umfaßt einen um eine Welle schwenkbaren Arm, der mit einem an jedem Ende angeordneten, nach außen gerichteten Stift versehen ist, wobei die Stifte mit den Ausnehmungen in Eingriff gebracht werden können. Wenn der Arm in eine Lage geschwenkt ist, in der der eine Stift mit der Ausnehmung des einen Einstellrads des einen Verdampfers im Eingriff ist und diesen Verdampfer verschließt, kann der andere Verdampfer in eine gewünschte Lage eingestellt werden und umgekehrt. Eine solche Verriegelungsanordnung ist ausschließlich auf zwei Verdampfer beschränkt.

Die Druckschriften EP-A-0 376 649 und US-A-4 434 790 offenbaren weitere Verriegelungsanordnungen mit mehreren nebeneinander angeordneten Verdampfern für ein Narkosegerät.

Der Erfindung liegt die Aufgabe zugrunde, eine Verriegelungsanordnung der eingangs genannten Art, insbesondere in Verbindung mit einem Verdampferkarussell zu schaffen, die in Verbindung mit einer Anzahl Verdampfer auf eine einfache und für den Benutzer sichere Weise sicherstellt, daß stets lediglich ein Verdampfer eingestellt werden kann.

Diese Aufgabe wird erfindungsgemäß dadurch gelöst, indem die Mittel als mechanischen Mittel ausgebildet sind, die so angeordnet sind, daß sie sich kreuzen, wobei die mechanischen Mittel im Kreuzungsbereich derart ausgebildet sind, daß, wenn ein Verdampfer aktiviert wird, das mechanische Mittel für diesen Verdampfer verschoben wird und dabei die übrigen mechanischen Mittel in einer Lage verschließt, in der ein Aktivieren der weiteren Verdampfer verhindert wird.

In Verbindung damit, daß jeder Verdampfer ein Ventil umfaßt, das den Gasfluß durch den jeweiligen Verdampfer steuert, wird die genannte Aufgabe gelöst, indem die mechanischen Mittel weiterhin die Ventile derart beeinflussen, daß, wenn ein Verdampfer aktiviert wird, das dazugehörige Ventil öffnet und gleichzeitig die zu den übrigen Verdampfern zugehörigen Ventile geschlossen werden.

In einer vorteilhaften Weiterbildung der Erfindung wird vorgeschlagen, daß die Verdampfer mit den dazugehörenden Ventilen genau gegenüber einander angeordnet sind. Hierdurch kann die Form der mechanischen Mittel verhältnismäßig einfach und dadurch in der Herstellung billig sein.

Nach der Erfindung besteht das mechanische Mittel aus mindestens einem Schieber. Damit man in der Produktion die Verriegelungsanordnung leichter montieren kann, kann es von Vorteil sein, wenn das mechanische Mittel aus zwei Schiebern besteht, die achsfluchtend zueinander liegen.

Im Hinblick auf eine vorteilhafte Weiterbildung der Erfindung wird vorgeschlagen, daß die Schieber derart geformt sind, daß sie im Kreuzungsbereich ineinander einflechtbar sind.

Hierdurch wird erreicht, daß die Enden der verschiedenen Schieber etwa in der gleichen Ebene liegen können, was bedeutet, daß sämtliche Verdampfer und Ventile in derselben Höhe am Gasverteiler angebracht werden können.

In einer weiteren vorteilhaften Ausbildung der Erfindung, bei der mindestens drei Verdampfer am Gasverteiler angeordnet sind, wird vorgeschlagen, daß die Schieber mit jeweils einem trapezförmigen Absatz versehen sind, der in einer Ebene im Kreuzungsbereich angebracht ist und dessen stumpfes Ende gegen einen fiktiven zentralen Punkt im Kreuzungsbereich gerichtet ist und daß, wenn ein Verdampfer zum Aktivieren gebracht wird, der Schieber für diesen Verdampfer in seiner Längsrichtung und damit der Absatz gegen den zentralen Punkt in eine Lage verschoben wird, in der dieser Absatz die übrigen Absätze verschließt und damit verhindert, daß die übrigen Schieber in eine aktive Verdampferlage verschoben werden können. Hierdurch ist eine Verriegelungsanordnung gegeben, die auch in Verbindung mit drei oder mehreren Verdampfern auf eine für den Benutzer sichere Weise sicherstellt, daß nur jeweils ein Verdampfer geregelt werden kann.

Weitere Vorteile und Einzelheiten ergeben sich aus den Unteransprüchen.

Die Erfindung ist nachfolgend anhand eines in den Zeichnungen dargestellten Ausführungsbeispieles näher erläutert. Es zeigen:
- FIG 1: schematisch ein Narkosegerät mit einem Verdampferkarussell,
- FIG 2: das Verdampferkarussell nach der FIG 1 in einer perspektivischen Ansicht, mit einer Verriegelungsanordnung nach der Erfindung,
- FIG 3: ein Teil der Verriegelungsanordnung nach der Erfindung in einer Darstellung in aufgelösten Einzelteilen,
- FIG 4: eine Verriegelungsanordnung nach FIG 3 in einer zusammengebauten Darstellung und
- FIG 5 und 6: Draufsichten der Verriegelungsanordnung nach den FIG 2, 3 und 4 in verschiedenen Lagen.

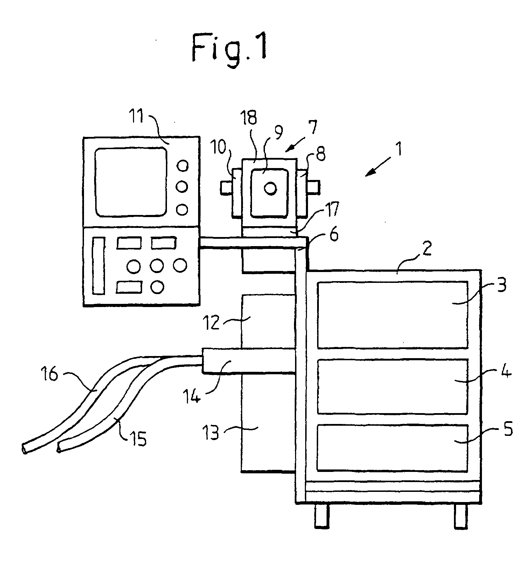

In der FIG 1 ist ein Narkosegerät 1 gezeigt, das einen Wagen 2 mit einer pneumatischen Einheit 3 für eine gasregulierende Ausrüstung, eine elektronische Einheit 4 für eine elektronische Ausrüstung sowie, falls eine Stromunterbrechung entsteht, eine Sicherungseinheit 5 für eine Batterie, umfaßt. Am Wagen 2 ist ein Gas- und Signalleitungen enthaltendes Stativ 6 vorgesehen. An dem Stativ ist ein Verdampferkarussell 7 angeordnet. Das Verdampferkarussell 7 ist mit einem ersten Verdampfer 8, einem zweiten Verdampfer 9 sowie einem dritten Verdampfer 10 versehen, die das gleiche oder verschiedene Narkosemittel beinhalten können.

Eine Kontroll- und Ueberwachungseinheit ist am Stativ 6 angeordnet. Außerdem ist eine Balgeinheit 12 und ein Absorber 13 an einer Patientenkassette 14 am Stativ 6 angeschlossen. Von der Patientenkassette 14 verläuft ein Inspirationsschlauch 15 und ein Exspirationsschlauch 16, die dazu vorgesehen sind, über ein Y-Stück oder Ähnliches an den Patienten, der einer Narkose unterzogen wird, angeschlossen zu werden.

Das Verdampferkarussell 7 umfaßt eine Gasversorgungseinheit 17 und eine Halterung bzw. einen Gasverteiler 18. Die Halterung bzw. der Gasverteiler 18 ist, zusammen mit den Verdampfern 8, 9, 10, gegenüber der Gasversorgungseinheit 17 drehbar angeordnet.

In dem Ausführungsbeispiel wird die Verriegelungsanordnung nach der Erfindung in Verbindung mit Verdampfern mit dazugehörenden Ventilen beschrieben.

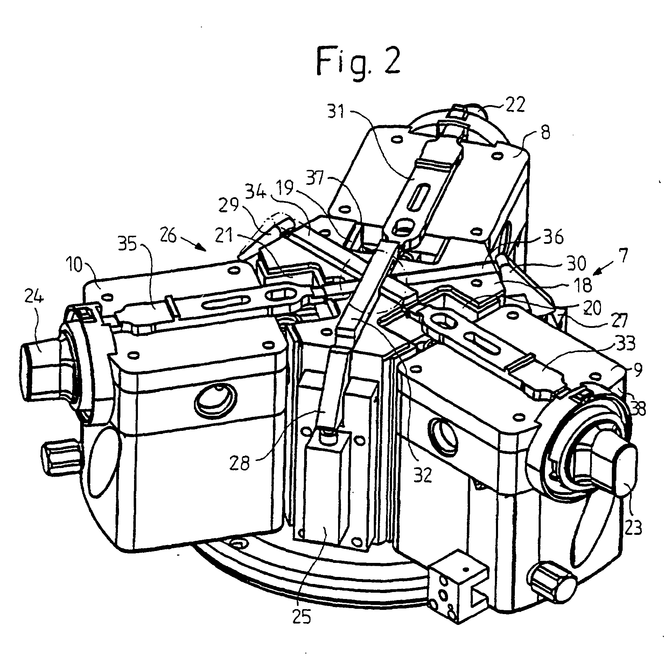

In der FIG 2 ist das Verdampferkarussell 7 mit der Halterung bzw. dem Gasverteiler 18 in einem im Vergleich zur FIG 1 größeren Maßstab gezeigt. In dieser FIG sind außerdem die Verdampfer und die Halterung ohne Deckel dargestellt, um den Aufbau der Verriegelungsanordnung nach der Erfindung zeigen zu können. Die Verdampfer 8,9, 10 sind an hierzu angepaßte Ausnehmungen 19, 20, 21, die an der Halterung 18 symmetrisch angebracht sind, anschließbar. Genau gegenüber jedem Verdampfer 8, 9, 10 ist ein jedem Verdampfer dazugehörendes Ventil 25, 26,27 angeordnet, das in der Halterung 18 befestigt und dafür vorgesehen ist, den Gasfluß durch den jeweiligen Verdampfer 8, 9, 10 weiter an einem an dem Narkosegerät 1 angeschlossenen, hier nicht dargestellten Patienten, zu steuern. Im Dokument EP-A-0 860 176 ist das Gasleitungssystem des Narkosegeräts und damit auch das Gasleitungssystem in der Halterung 18 zwischen den Verdampfern 8, 9, 10 und den Ventilen 25, 26, 27 ausführlich beschrieben.

Jedes Ventil 25, 26, 27 ist mit einem federgespannten Hebel 28, 29, 30 versehen, wobei das jeweilige Ventil 25, 26, 27 in der einen Lage des Hebels 28, 29, 30 offen und in der anderen Lage des Hebels 28, 29, 30 geschlossen ist. Jeder Verdampfer 8, 9, 10 ist über Schieber mit jeweils einem Hebel 28, 29, 30 der dazugehörenden Ventile 25, 26, 27 verbunden. So ist der Verdampfer 8 über Schieber 31, 32 mit dem Hebel 28 des Ventils 25, der Verdampfer 9 über Schieber 33, 34 mit dem Hebel 29 des Ventils 26 und der Verdampfer 10 über Schieber 35, 36 mit dem Hebel 30 des Ventils 27 verbunden. Die paarweise genannten Schieber 31, 32; 33, 34; und 35, 36 sind achsfluchtend zueinander angeordnet. Die Schieber 32, 34, 36 kreuzen sich in einem Kreuzungsbereich 37, wobei die Schieber 32, 34, 36 so geformt sind, daß sie im Kreuzungsbereich 37 ineinander einflechtbar sind. Die Schieber 32, 34, 36 sind außerdem im Kreuzungsbereich 37 derart ausgebildet, daß, wie später näher beschrieben, ein Aktivieren von zwei oder mehr Verdampfern verhindert wird.

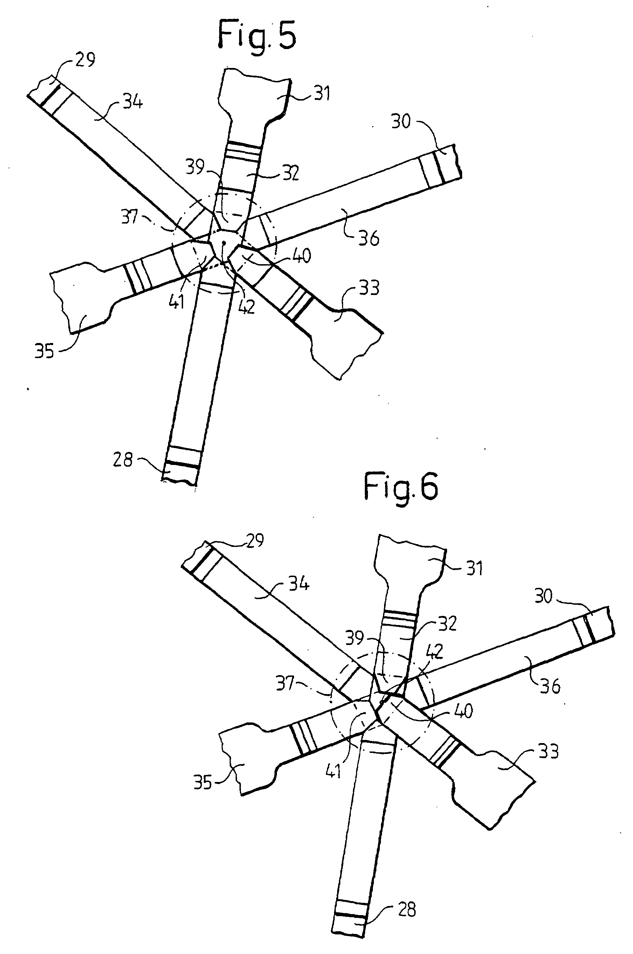

Jeder Verdampfer 8, 9, 10 ist mit einem Einstellrad 22, 23, 24 zum Aktivieren der jeweiligen Verdampfer 8, 9, 10 und Einstellen der gewünschten Gaskonzentration ausgerüstet. Beim Aktivieren z.B. des Verdampfers 9 wird das Einstellrad 23 gegen das Verdampfergehäuse 38 gedrückt und aktiviert dabei den Schieber 33 und damit den Schieber 34, so daß sie in ihre Längsrichtungen verschoben derart werden, daß sie den Hebel 29 beeinflußen in eine Lage zu kippen, die mit Hilfe der gestrichelten Konturen gezeigt wird. In dieser Lage ist das Ventil 26, das in dieser FIG nicht zu sehen ist, offen. Die Gaskonzentration wird durch Drehen des Einstellrades 23 in die gewünschte Lage eingestellt. Beim Drehen wird die eingedrückte Lage des Einstellrades 23 mittels bekannter und daher nicht näher beschriebener Mittel beibehalten. In dieser Lage verschließt der Schieber 34 die übrigen Schieber 32 und 36, so daß, wie es in Verbindung mit den FIG 5 und 6 näher beschrieben wird, die Verdampfer 8 und 10 nicht aktiviert werden können. Wenn das Einstellrad 23 in eine Null-Lage zurückgedreht wird, werden die Schieber 33, 34 und das Einstellrad 23 mit Hilfe der Federkraft des federgespannten Hebels 29 in ihren ursprünglichen Lagen zurückgedrückt. Auch der Hebel 29 geht in seine ursprüngliche Lage zurück und schließt dadurch das Ventil 26. Auf beschriebene Weise erfolgt beim Aktivieren der übrigen Verdampfer 8 und 10 ein Verschieben der jeweils dazugehörenden Schieber und Hebel, wobei nur ein Verdampfer gleichzeitig aktiviert werden kann.

Die obere Fläche der Halterung 18 ist mit Nuten versehen, in die die Schieber 32, 34, 36 in ihren jeweiligen Richtungen verschiebbar angeordnet sind. Die Nuten, die in der FIG nicht gezeigt sind, dienen dazu, die Längsverschiebung der Schieber 32, 34, 36 zu stabilisieren. Die Schieber 31, 33 und 35 sind in Nuten, die in den bereits erwähnten, in der FIG nicht gezeigten Verdampferdeckeln angeordnet sind, verschiebbar.

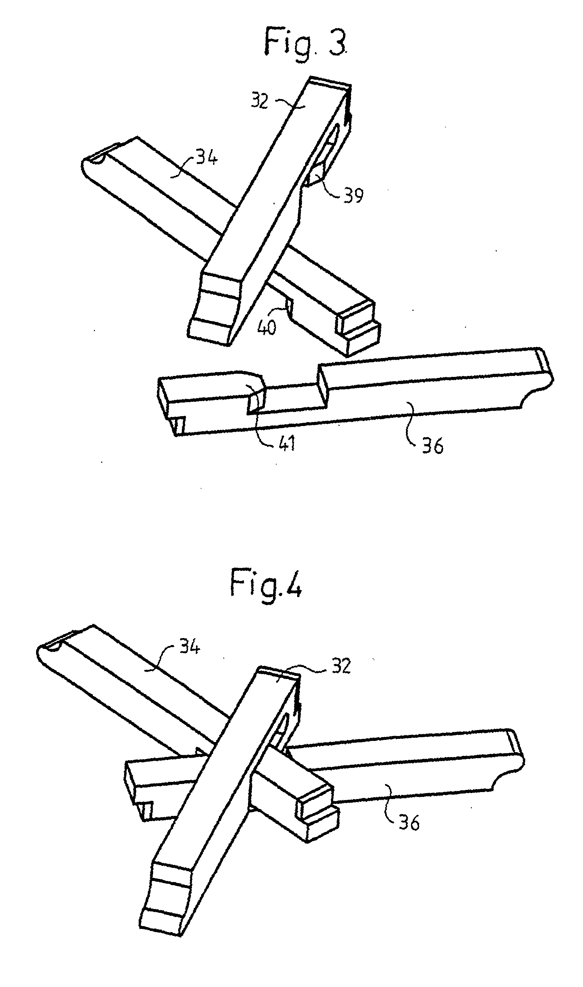

In der FIG 3 sind die Schieber 32, 34,36 in einer auseinandergenommenen Lage gezeigt. In dieser FIG ist es deutlich dargestellt, daß die Schieber im Kreuzungsbereich 37 derart geformt sind, daß sie ineinander einflechtbar sind. Jeder Schieber ist mit einem Absatz versehen. So ist der Schieber 32 mit einem Absatz 39, der Schieber 34 mit einem Absatz 40 und der Schieber 36 mit einem in dieser FIG deutlich dargestellten Absatz 41, versehen.

In der FIG 4 sind die Schieber 32, 34,36 in einer eingeflochtenen Lage gezeigt und damit, wie in der FIG 1 so angeordnet, daß die Absätze 39, 40, 41 (FIG 3) in einer Ebene im Kreuzungsbereich 37 (FIG 2) angebracht sind.

In den FIG 5 und 6 ist die Ebene der Verriegelungsanordnung gezeigt, in der die Absätze 39, 40, 41 wirken. Wie aus den FIG hervorgeht, sind die Absätze 39, 40, 41 trapezförmig ausgebildet. In der FIG 5 ist gezeigt, daß die stumpfen Enden der Absätze 39, 40, 41 gegen einen fiktiven zentralen Punkt 42 im Kreuzungsbereich 37 gerichtet sind. In dieser FIG 5 weisen die Schieber 32, 34, 36 mit den dazugehörenden Absätzen 39, 40, 41 eine Lage auf, in der keiner der Verdampfer 8, 9, 10 aktiviert worden ist.

In der FIG 6 ist eine Lage der Schieber 34 gezeigt, in der der Verdampfer 9 auf beschriebene Weise aktiviert wird. Der Schieber 33 und damit der Schieber 34 werden dann in ihren Längsrichtungen und der Absatz 40 in Richtung des zentralen Punktes 42 verschoben. In dieser einen Endlage blockiert der Absatz 40 die übrigen Absätze 39, 41 und verhindert damit auch, daß die übrigen Schieber 32, 35 und 39 in eine aktive Verdampfer-/Ventillage verschoben werden können.

Die beschriebenen mechanischen Mittel zwischen den Verdampfern und den Ventilen brauchen nicht notwendiger Weise zwei nacheinander angeordnete Schieber zu umfassen, sondern können durch einen einzigen entsprechenden langen Schieber ersetzt werden.

Bis jetzt wurde lediglich der Hergang in Verbindung mit einem Aktivieren des Verdampfers 9 gezeigt und beschrieben. Der Hergang ist beim Aktivieren der Verdampfer 8 und 10 selbstverständlich derselbe.

Im Rahmen der Erfindung kann die Halterung 18 des Verdampferkarussells 7 für sowohl zwei als auch für mehr als drei Verdampfer ausgebildet sein. Die Verriegelungsanordnung nach der Erfindung kann selbstverständlich in einem solchen Fall, in dem Ventile nicht beeinflußt werden, verwendet werden. Das Wesentliche mit der Verriegelungsanordnung ist es, daß hier stets lediglich ein Verdampfer gleichzeitig aktiviert werden kann.

## Patentansprüche

1. Verriegelungsanordnung für Verdampfer (8, 9, 10) bei einem Narkosegerät (1), bei dem das Gerät (1) mindestens zwei Verdampfer (8, 9, 10) zum Einstellen einer gewünschten Gaskonzentration umfaßt, wobei die Verriegelungsanordnung Mittel aufweist, die dazu vorgesehen sind zu verhindern, daß mehr als ein Verdampfer (8, 9, 10) gleichzeitig aktiviert wird, wobei die Verdampfer (8, 9, 10) mit einem Gasverteiler (18) verbindbar sind, **dadurch gekennzeichnet, daß** die Mittel als mechanischen Mittel (31 bis 36) ausgebildet sind, die so angeordnet sind, daß sie sich kreuzen, wobei die mechanischen Mittel (31 bis 36) im Kreuzungsbereich (37) derart ausgebildet sind, daß, wenn ein Verdampfer (8, 9, 10) aktiviert wird, das mechanische Mittel (31 bis 36) für diesen Verdampfer (8, 9, 10) verschoben wird und dabei die übrigen mechanischen Mittel (31 bis 36) in einer Lage verschließt, in der ein Aktivieren der weiteren Verdampfer (8, 9, 10) verhindert wird.

2. Verriegelungsanordnung nach Anspruch 1, wobei jeder Verdampfer (8, 9, 10) ein Ventil (25, 26, 27) umfaßt, das den Gasfluß durch den jeweiligen Verdampfer (8, 9, 10) steuert, **dadurch gekennzeichnet, daß** die mechanischen Mittel (31 bis 36) weiterhin die Ventile (25, 26, 27) derart beeinflussen, daß, wenn ein Verdampfer (8, 9, 10) aktiviert wird, das dazugehörige Ventil (25, 26, 27) öffnet und gleichzeitig die zu den übrigen Verdampfern (8, 9, 10) zugehörigen Ventile (25, 26, 27) geschlossen werden.

3. Verriegelungsanordnung nach Anspruch 2, **dadurch gekennzeichnet, daß** die Verdampfer (8, 9, 10) mit den dazugehörenden Ventilen (25, 26, 27) genau gegenüber einander angeordnet sein können.

4. Verriegelungsanordnung nach einem der Ansprüche 1 bis 3, **dadurch gekennzeichnet, daß** das mechanische Mittel (31 bis 36) aus mindestens einem Schieber besteht.

5. Verriegelungsanordnung nach einem der Ansprüche 1 bis 4, **dadurch gekennzeichnet, daß** die Schieber (32, 34, 36) derart geformt sind, daß sie im Kreuzungsbereich (37) ineinander einflechtbar sind.

6. Verriegelungsanordnung nach einem der Ansprüche 1 bis 5, **dadurch gekennzeichnet, daß** die Schieber (32, 34, 36) mit jeweils einem Absatz (39, 40, 41) versehen sind und diese Absätze die in einer Ebene im Kreuzungsbereich (37) angebracht sind und wobei deren Enden gegeneinander gerichtet sind.

7. Verriegelungsanordnung nach einem der Ansprüche 1 bis 6, bei der mindestens drei Verdampfer am Gasverteiler angebracht sind, **dadurch gekennzeichnet, daß** die Schieber (32, 34, 36) mit jeweils einem trapezförmigen Absatz (39, 40, 41) versehen sind, der in einer Ebene im Kreuzungsbereich (37) angebracht ist und dessen stumpfes Ende gegen einen fiktiven zentralen Punkt (42) im Kreuzungsbereich (37) gerichtet ist und daß, wenn ein Verdampfer (8, 9, 10) zum Aktivieren gebracht wird, der Schieber (32, 34, 36) für diesen Verdampfer (8, 9, 10) in seiner Längsrichtung und damit der Absatz (39, 40, 41) gegen den zentralen Punkt (42) in eine Lage verschoben wird, in der dieser Absatz (39, 40, 41) die übrigen Absätze (39, 40, 41) verschließt und damit verhindert, daß die übrigen Schieber (32, 34, 36) in eine aktive Verdampferlage verschoben werden können.

8. Verriegelungsanordnung nach einem der Ansprüche 1 bis 7, **dadurch gekennzeichnet, daß** die Verdampfer (8, 9, 10) am Gasverteiler (18) symmetrisch angebracht sein können.

## Claims

1. Interlock arrangement for vaporisers (8, 9, 10) at a narcosis apparatus (1), which apparatus (1) comprises at least two vaporisers (8, 9, 10) for setting a desired gas concentration, whereby the interlock arrangement comprises means arranged to prevent more than one vaporiser (8, 9, 10) from being activated at the same time, whereby the vaporiser (8, 9, 10) is connectable with a gas distributor (18), **characterised in that** the means are formed as mechanical means (31 to 36) arranged to cross each other, whereby the mechanical means (31 to 36) in the crossing region (37) are so formed that when a vaporiser (8, 9, 10) is activated the mechanical means (31 to 36) for this vaporiser (8, 9, 10) is displaced and thereby locks the other mechanical means (31 to 36) in a position where activation of the other vaporisers (8, 9, 10) is prevented.

2. Interlock arrangement according to claim 1, whereby each vaporiser (8, 9, 10) comprises a valve (25, 26, 27) which controls the gas flow through each corresponding vaporiser (8, 9, 10), **characterised in that** the mechanical means (31 to 36) further influence the valve (25, 26, 27) so that when a vaporiser (8, 9, 10) is activated, the corresponding valve (25, 16, 27) is opened and at the same time the valves (25, 16, 27) corresponding to the other vaporisers (8, 9, 10) are closed.

3. Interlock arrangement according to claim 2, **characterised in that** the vaporisers (8, 9, 10) with their corresponding valves (25, 16, 17) can be arranged opposite each other.

4. Interlock arrangement according to any of claims 1 to 3, **characterised in that** the mechanical means (31 to 36) consists of at least one slide.

5. Interlock arrangement according to any of claims 1 to 4, **characterised in that** the slides (32, 34, 36) are formed so that they are intertwinable with each other in the crossing region (37).

6. Interlock arrangement according to any of claims 1 to 5, **characterised in that** the slides (32, 34, 36) are provided with one shoulder (39, 40, 41) each, which shoulders (39, 40, 41) are arranged in one plane in the crossing region (37) and whose ends are directed against each other.

7. Interlock arrangement according to any of claims 1 to 6, wherein at least three vaporisers are arranged at the gas distributor, **characterised in that** the slides (32, 34, 36) are provided with one trapezoidal shoulder (39, 40, 41) each, which are arranged in one plane in the crossing region (37) and whose blunt ends are directed towards a fictitious centre point (42) in the crossing region (37) and **in that** when a vaporiser (8, 9, 10) is brought to be activated, the slide (32, 34, 36) for this vaporiser (8, 9, 10) is displaced longitudinally and thereby also the shoulder (39, 40, 41) towards the centre point (42) to a position in which this shoulder (39, 40, 41) blocks the other shoulders (39, 40, 41) and thereby prevents the other slides (32, 34, 36) to be displaced towards an active vaporiser position.

8. Interlock arrangement according to any of claims 1 to 7, **characterised in that** the vaporisers (8, 9, 10) can be arranged symmetrically at the gas distributor (18).

## Revendications

1. Dispositif de verrouillage pour des vaporisateurs (8, 9, 10) dans un appareil (1) d'anesthésie, dans lequel l'appareil (1) comprend au moins deux vaporisateurs (8, 9,10) pour le réglage d'une concentration souhaitée de gaz, le dispositif de verrouillage ayant des moyens destinés à empêcher que plus d'un vaporisateur (8, 9, 10) soit activé en même temps, les vaporisateurs (8, 9, 10) pouvant communiquer avec un répartiteur (18) de gaz , **caractérisé en ce que** les moyens sont constitués en moyens (31 à 36) mécaniques qui sont disposés de manière à se croiser, les moyens (31 à 36) mécaniques étant constitués dans la zone (37) d'intersection de façon à ce que, lorsqu'un vaporisateur (8, 9, 10) est activé, le moyen (31 à 36) mécanique pour ce vaporisateur (8, 9, 10) est déplacé et ferme ainsi les autres moyens (31 à 36) mécaniques en les mettant dans une position dans laquelle une activation des autres vaporisateurs (8, 9, 10) est empêchée.

2. Dispositif de verrouillage suivant la revendication 1, dans lequel chaque vaporisateur (8, 9,10) comprend une vanne (25, 26, 27) qui commande le flux de gaz passant dans le vaporisateur (8, 9, 10) respectif, **caractérisé en ce que** les moyens (31 à 36) mécaniques influencent en outre les vannes (25, 26, 27) de façon à ce que, lorsqu'un vaporisateur (8, 9, 10) est activé, la vanne (25, 26, 27) associée est ouverte et en même temps les vannes (25, 26, 27) associées aux autres vaporisateurs (8, 9, 10) sont fermées.

3. Disposition de verrouillage suivant la revendication 2, **caractérisé en ce que** les vaporisateurs (8, 9,10) avec les vannes (25, 26, 27) associées peuvent être disposés de manière précise les uns par rapport aux autres.

4. Dispositif suivant l'une des revendications 1 à 3, **caractérisé en ce que** le moyen (31 à 36) mécanique est constitué d'un coulisseau.

5. Dispositif suivant l'une des revendications 1 à 4, **caractérisé en ce que** les coulisseaux (32, 34, 36) sont conformés de manière à pouvoir être insérés les uns dans les autres dans la zone (37) d'intersection.

6. Dispositif suivant l'une des revendications 1 à 5, **caractérisé en ce que** les coulisseaux (32, 34, 36) sont munis d'un talon (39, 40, 41) respectif et ces talons sont disposés dans un plan dans la zone (37) d'intersection et leur extrémité sont dirigées en sens opposé.

7. Dispositif suivant l'une revendications 1 à 6, dans lequel il est prévu au moins trois vaporisateurs sur le répartiteur de gaz, **caractérisé en ce que** les coulisseaux (32, 34, 36) sont munis respectivement d'un talon (39, 40, 41) trapézoïdal qui est disposé dans un plan dans la zone (37) d'intersection et dont la petite base est dirigée vers un point (42) central fictif dans la zone (37) d'intersection et **en ce que**, lorsqu'un vaporisateur (8, 9, 10) est mis en activité, le coulisseau (32, 34, 36) de ce vaporisateur (8, 9, 10) est déplacé dans sa direction longitudinale et ainsi le talon (39, 40, 41) est déplacé vers le point (41) central pour venir dans une position dans laquelle ce talon (39, 40, 41) ferme les autres talons (39, 40, 41) et empêche ainsi que les autres coulisseaux (32, 34, 36) puissent être déplacés pour venir dans une position active du vaporisateur.

8. Dispositif suivant l'une des revendications 1 à 7, **caractérisé en ce que** les vaporisateurs (8, 9, 10) peuvent être disposés symétriquement sur le répartiteur (18) de gaz.
